# DEMANDE DE BREVET EUROPEEN

(11) **EP 2 168 563 A2**
(43) Date de publication de la demande: **31.03.2010**
(21) Numéro de dépôt: 09171685.2
(22) Date de dépôt: 29.09.2009
(51) Int. Cl.: A61K 8/06, A61K 8/81, A61K 8/92, A61K 8/55, A61Q 1/10, A61K 8/90

(54) **Composition de maquillage des cils et ensemble de conditionnement**

(30) Priorité: 30.09.2008 FR 0856546
(71) Demandeur: L'Oréal, 75008 Paris (FR)
(72) Inventeur: Dumousseaux, Christophe, 92160, Antony (FR); Le Merrer, Carole, 92120, Montrouge (FR)
(74) Mandataire: Goudet, Sylvain

(57) **Abrégé**

La présente invention a pour objet une composition cosmétique de maquillage ou de soin non thérapeutique des fibres kératiniques comprenant une phase aqueuse continue, au moins un composé choisi parmi les copolymères comprenant au moins un monomère alcène, au moins une cire polaire et au moins un tensioactif particulier.

## Description

La présente invention a pour objet une composition cosmétique de maquillage ou de soin non thérapeutique des fibres kératiniques (telles que les cils, les sourcils, les cheveux) comprenant une phase aqueuse continue, au moins un composé choisi parmi les copolymères comprenant au moins un monomère alcène, au moins une cire polaire, et au moins un tensioactif particulier.

Elle se présente en particulier sous la forme d'un mascara, d'un produit pour les sourcils. Plus spécialement, l'invention porte sur un mascara.

Par mascara, on entend une composition destinée à être appliquée sur les fibres kératiniques : il peut s'agir d'une composition de maquillage des fibres kératiniques, une base de maquillage des fibres kératiniques ou base-coat, une composition à appliquer sur un mascara, dite encore top-coat, ou bien encore une composition de traitement cosmétique des fibres kératiniques. Le mascara est plus particulièrement destiné aux fibres kératiniques d'êtres humains, mais également aux faux-cils.

Les compositions de maquillage pour les yeux, encore appelées « mascaras » pour les cils, sont généralement constituées d'une cire ou mélange de cires dispersée(s) à l'aide d'au moins un tensioactif dans une phase aqueuse contenant, par ailleurs, des polymères et des pigments.
C'est généralement à travers le choix qualitatif et quantitatif des cires et polymères que sont ajustées les spécificités d'application recherchées pour les compositions de maquillage, comme par exemple leur fluidité, leur pouvoir couvrant et/ou leur pouvoir recourbant. Ainsi, il est possible de réaliser diverses compositions qui, appliquées notamment sur les cils, induisent des effets variés de type allongement, recourbement et/ou épaississement (effet chargeant).

La présente invention vise plus particulièrement à proposer une composition utile pour réaliser un maquillage épais des fibres kératiniques et notamment des cils, dit encore maquillage chargeant. Au sens de la présente invention, le terme fibres kératiniques couvre les cheveux, les cils et les sourcils et s'étend également aux postiches et faux-cils synthétiques.

Avec les compositions de maquillage actuellement disponibles, cet effet est généralement obtenu par superposition de plusieurs couches de la composition de maquillage au niveau des fibres kératiniques et plus particulièrement des cils. Par ailleurs, dans le cas particulier des cils, cet effet est très souvent associé à une agglomération de plusieurs cils entre eux, c'est-à-dire à une non individualisation des cils.
Pour ce faire, certaines compositions de maquillage ont été proposées, qui sont suffisamment concentrées en matière sèche pour charger significativement le cil dès sa première mise en contact avec lesdites compositions. Cependant, de telles compositions peuvent devenir trop épaisses à l'application et ne plus présenter la déformabilité nécessaire à leur application homogène sur toute la surface des cils.
En outre, de telles compositions ne sont pas idéales en termes d'agrément et de confort à l'application : elles donnent parfois un côté freinant et sec lors de l'application.
Pour des raisons évidentes, il serait avantageux d'obtenir une composition présentant à la fois un effet chargeant immédiat, une consistance suffisante, et un bon glissant sans effet de sécheresse ou de frein à l'application, notamment avec une brosse.

Les inventeurs ont découvert que les propriétés décrites ci-dessus peuvent être obtenues en utilisant une composition comprenant une phase aqueuse continue, au moins un composé choisi parmi les copolymères comprenant au moins un monomère alcène, au moins une cire polaire et au moins un tensioactif ionique présent dans la phase aqueuse sous forme de sel.

De façon plus précise, l'invention a pour objet une composition cosmétique de maquillage ou de soin non thérapeutique des fibres kératiniques comprenant une phase aqueuse continue, au moins un composé choisi parmi les copolymères comprenant au moins un monomère alcène, au moins une cire polaire et au moins un tensioactif ionique présent dans la phase aqueuse sous forme de sel.

La présente invention a également pour objet un procédé cosmétique de maquillage ou de soin non thérapeutique des fibres kératiniques comprenant l'application sur les fibres kératiniques d'une composition selon l'invention.

La présente invention a également pour objet un ensemble de conditionnement d'une composition de maquillage et/ou de soin des fibres kératiniques, notamment des cils ou des sourcils, comprenant :
i) un récipient délimitant au moins un compartiment, et
ii) une composition selon l'invention disposée à l'intérieur dudit compartiment.

La composition comprend un milieu physiologiquement acceptable, c'est-à-dire un milieu non toxique et susceptible d'être appliqué sur les fibres kératiniques, telles que les cils, les sourcils et les cheveux d'êtres humains, notamment compatible avec la zone oculaire.

### Copolymères comprenant au moins un monomère alcène, en particulier les copolymères à base d'éthylène

Par « monomère alcène », on entend un monomère hydrocarbure dont la chaîne carbonée, linéaire ou ramifiée, présente une double liaison carbone-carbone. L'alcène selon l'invention est donc dépourvu d'hétéroatome.
Par « copolymère », on entend un polymère comprenant 2 monomères différents.

De tels composés peuvent être choisis parmi :
- les copolymères d'alcène et d'acétate de vinyle, de préférence les copolymères d'éthylène et d'acétate de vinyle.
   De tels copolymères ont l'avantage de combiner un faible point de fusion, une bonne compatibilité avec les cires hydrocarbonées, et une bonne adhérence sur différents supports, notamment les fibres kératiniques.
   On peut utiliser les copolymères suivants : acétate de vinyle/octadécène, ou acétate de vinyle/dodécène-1.
   On utilise de préférence les copolymères d'éthylène et d'acétate de vinyle comprenant de préférence entre 5 et 50% en poids d'acétate de vinyle par rapport au poids total du polymère, de préférence entre 10 et 45% en poids, de préférence entre 20 et 40% en poids. L'augmentation de la teneur en monomère acétate de vinyle dans le copolymère permet d'augmenter l'adhérence sur les fibres kératiniques.
   Comme exemple de copolymères éthylène/acétate de vinyle, on peut citer ceux qui sont commercialisés sous la dénomination ELVAX par la société Du Pont de Nemours et en particulier les composés Elvax 40W, Elvax 140W, Elvax 200W, Elvax 205W, Elvax 210W et Elvax 310.
   On peut également citer les produits commercialisés sous la dénomination EVATANE par la société Arkema tels que l'Evatane 28-800.
- les copolymères d'α-oléfine linéaire ou ramifiée, en particulier d'α-oléfine en C₂-C₁₆ et mieux en C₂-C₁₂. De préférence, ces copolymères sont des bi- ou terpolymères et tout particulièrement des bipolymères.
   Parmi les bipolymères recommandés pour les compositions de l'invention, on peut citer les bipolymères d'éthylène et d'α-oléfine en C₄-C₁₆, de préférence en C₄-C₁₂ et les bipolymères de propylène et d'α-oléfine en C₄-C₁₆, de préférence en C₄-C₁₂. De préférence encore, l'α-oléfine est choisie parmi le butène-1, le pentène-1, l'hexène-1, l'octène-1, le nonène-1, le décène-1, l'undécène-1, le dodécène-1, le 3,5,5-triméthylhexène-1, le 3-méthylpentène-1, et le 4-méthylpentène-1.
   Parmi ces monomères, le butène-1 et l'octène-1 sont particulièrement préférés.
   Les bipolymères recommandés sont les élastomères ayant un taux de cristallinité allant de 10 à 35 %.
   Ces bipolymères sont préférentiellement synthétisés par catalyse métallocène.
   De tels bipolymères sont commercialisés par la Société DOW CHEMICAL sous les dénominations commerciales « AFFINITY » (plastomères) et par la Société Dupont de Nemours sous la dénomination « ENGAGE » (élastomères).
   Des bipolymères éthylène-butène sont commercialisés par la Société EXXON sous l'appellation commerciale « EXACT RESINS » et par la société ELENAC sous l'appellation commerciale « LUFLEXEN ».
   Parmi les terpolymères, on peut citer les terpolymères d'éthylène, de propylène et d'αoléfine en C₄-C₁₆, de préférence C₄-C₁₂.
   Dans ces terpolymères, les teneurs en α-oléfine en C₄-C₁₆ sont comme indiquées précédemment et les α-oléfines préférées sont le butène, l'hexène et l'octène.
   Les copolymères préférés, décrits dans la demande EP-A-1 034 776, peuvent en particulier être les copolymères d'éthylène et d'octène tels que par exemple les produits commercialisés sous la référence « AFFINITY» par la société Dow Plastics comme par exemple l'AFFINITY GA 1900 GA 1950 ;
- les copolymères d'éthylène ou de propylène et d'une cyclooléfine, en particulier les bipolymères.
   Généralement, la teneur en cyclooléfine des copolymères est inférieure à 20% en mole.
   Parmi les cyclooléfines utilisables, on peut citer le cyclobutène, le cyclohexène, le cyclooctadiène, le norbornène, le diméthano-octahydronaphtalène (DMON), l'éthylidène norbornène, le vinyl norbornène et le 4-vinylcyclohexène.
   Les copolymères recommandés de cette classe sont les copolymères d'éthylène et de norbornène. La teneur en norbornène de ces copolymères est généralement inférieure à 18% en mole pour présenter le caractère cristallin requis et ces copolymères sont synthétisés par catalyse métallocène.
   Des copolymères éthylène/norbornène appropriés sont commercialisés par les Sociétés MITSUI PETROCHEMICAL ou MITSUI-SEKKA sous la dénomination commerciale « APPEL » et par la Société HOECHST-CELANESE sous la dénomination commerciale « TOPAS ».
- les copolymères à blocs (dibloc, tribloc, multibloc, radial, étoile) styrène et à blocs éthylène/alkylène en C3-C4.
   Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.
On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.
- et leurs mélanges.

Ces copolymères permettent d'épaissir la phase grasse, en particulier d'épaissir le mélange cireux à une température supérieure au point de fusion des cires. Cet épaississement de la phase grasse permet d'émulsionner plus efficacement en présence du tensioactif ionique présent dans la phase aqueuse. Cette viscosité élevée permet également d'avoir une très bonne dispersion des pigments à chaud dans la phase grasse et d'obtenir une très bonne intensité de couleur de la formulation.

Pour parvenir à un épaississement efficace de la phase grasse ces copolymères ont de préférence une masse moléculaire moyenne en poids Mw, exprimée en équivalent polystyrène, supérieure à 30000 Daltons, de préférence supérieure à 50000 Daltons, de façon encore plus préférentielle supérieure à 60000 Daltons.
La masse moléculaire moyenne en poids Mw est avantageusement choisie entre 30000 et 300000 Dalton, de préférence entre 50000 et 200000 Daltons.

Ces copolymères peuvent être utilisés seuls ou en mélange avec au moins un composé choisi parmi les résines dites tackifiantes telles que décrites dans le Handbook of Pressure Sensitive Adhesive, edited by Donatas Satas, 3rd ed., 1989, p. 609-619, les cires telles que décrites plus loin, et leurs associations. Les résines tackifiantes peuvent être notamment choisies parmi la colophane (correspondant au terme anglo-saxon « rosin »), les dérivés de colophane, les résines hydrocarbonées et leurs mélanges. On peut citer en particulier les résines hydrocarbonées indéniques telles que les résines issues de la polymérisation en proportion majoritaire de monomère indène et en proportion minoritaire de monomères choisis parmi le styrène, le méthylindène, le méthylstyrène et leurs mélanges. Ces résines peuvent éventuellement être hydrogénées. Elles peuvent présenter un poids moléculaire allant de 290 à 1150. Comme exemples de résines indéniques, on peut citer en particulier les copolymères indène/méthylstyrène/styrène hydrogéné commercialisées sous la dénomination « REGALITE » par la société Eastman Chemical, en particulier REGALITE R 1100, REGALITE R 1090, REGALITE R-7100, REGALITE R1010 HYDROCARBON RESIN, REGALITE R1125 HYDROCARBON RESIN.

Comme mélange à base de copolymère éthylène/acétate de vinyle, on peut citer par exemple les produits commercialisés sous la dénomination Coolbind par la société National Starch.

De préférence, ces copolymères sont présents dans la composition en une teneur en matières sèches supérieure ou égale à 0,5% en poids, de préférence supérieure ou égale à 0,7% en poids, mieux supérieur ou égal à 0,8% en poids et de préférence supérieure ou égale à 1% en poids par rapport au poids total de la composition.
De préférence, ces copolymères sont présents dans la composition en une teneur en matières sèches comprise entre 0.5% et 20% en poids par rapport au poids total de la composition, de préférence entre 1% et 10% en poids. Cette gamme de concentration permet d'obtenir un épaississement significatif de la phase grasse tout en restant facilement manipulable.

Ces copolymères peuvent se présenter sous forme pure ou être véhiculés dans une phase aqueuse ou une phase solvant organique.

### Phase aqueuse continue

La composition selon l'invention comprend un milieu aqueux, constituant une phase aqueuse, qui forme la phase continue de la composition. La phase aqueuse de la composition selon l'invention est ainsi une phase aqueuse continue.

Par composition à phase aqueuse « continue », on entend que la composition présente une conductivité, mesurée à 25°C, supérieure à 23 µS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄ et leur mélanges.
De préférence, la phase aqueuse (eau et éventuellement le solvant miscible à l'eau) est présente en une teneur au moins égale à 30% en poids par rapport au poids total de la composition, de préférence au moins égale à 40% en poids, de préférence au moins égale à 45 % en poids.
De préférence, la phase aqueuse (eau et éventuellement le solvant miscible à l'eau) est présente en une teneur comprise entre 30% et 90% en poids par rapport au poids total de la composition, de préférence comprise entre 40% et 80% en poids, de préférence comprise entre 45 % et 70% en poids.

### Tensioactif ionique présent dans la phase aqueuse sous forme de sel (ou sous forme salifiée)

Les compositions selon l'invention comprennent au moins un agent tensioactif ionique présent dans la phase aqueuse sous forme de sel. Un tel tensioactif est présent sous forme entièrement salifiée suite à une neutralisation avec une base forte comme la soude ou la potasse.
Ce tensioactif peut être présent notamment en une proportion allant de 0,1 à 20 %, et mieux de 0,3 % à 15 % en poids par rapport au poids total de la composition.

Par « tensioactif ionique présent dans la phase aqueuse sous forme de sel », on entend un tensioactif ionique présent sous forme ionisée dans le milieu aqueux continu (eau et éventuellement solvant miscible à l'eau).
Un tel tensioactif est notamment distinct des systèmes acide stéarique/triéthanolamine et acide stéarique/amino-2-méthyl-2-propane di-ol-1,3, qui implique le mélange *in situ* dans la composition de l'acide stéarique présent dans la phase grasse avec la triéthanolamine
ou l'amino-2-méthyl-2-propane di-ol-1,3 (sous forme non salifiée) présente dans la phase aqueuse, afin de former de façon partielle le stéarate de triéthanolamine ou le stéarate d'amino-2-méthyl-2-propane di-ol-1,3.
Un tel tensioactif permet d'émulsionner plus efficacement à haute température le copolymère comprenant au moins un monomère alcène.

Selon l'invention, on utilise généralement un émulsionnant ionique choisi de manière appropriée pour l'obtention d'une émulsion huile-dans-eau. En particulier, on peut utiliser un émulsionnant possédant à 25 °C une balance HLB (hydrophile-lipophile balance) au sens de GRIFFIN, supérieure ou égale à 8.
La valeur HLB selon GRIFFIN est définie dans J. Soc. Cosm. Chem. 1954 (volume 5), pages 249-256.
Ces agents tensioactifs peuvent être choisis parmi des agents tensioactifs anioniques, cationiques, amphotériques ou encore des émulsionnants tensioactifs. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques et amphotériques.
Les tensioactifs ioniques présents sous forme de sels dans la phase aqueuse selon l'invention sont choisis parmi :
a) les tensioactifs anioniques tels que :
   - les sels d'acides gras polyoxyéthylénés notamment ceux dérivant des sels alcalins, et leurs mélanges ;
   - les esters phosphoriques et leurs sels tels que le "DEA oleth-10 phosphate" (Crodafos N 10N de la société CRODA) ou le phosphate de monocétyle monopotassique (Amphisol K de Givaudan) ;
   - les sulfosuccinates tels que le "Disodium PEG-5 citrate lauryl sulfosuccinate" et le "Disodium ricinoleamido MEA sulfosuccinate" ;
   - les alkyléthersulfates tels que le lauryl éther sulfate de sodium ;
   - les iséthionates ;
   - les acylglutamates tels que le "Disodium hydrogenated tallow glutamate" (AMISOFT HS-21 R^{®} commercialisé par la société AJINOMOTO) et le sodium stearoyl glutamate (AMISOFT HS-11 PF^{®} commercialisé par la société AJINOMOTO) et leurs mélanges ;
   - les dérivés de soja comme le potassium soyate ;
   - les citrates, comme le Glyceryl stearate citrate (Axol C 62 Pellets de Degussa) ;
   - les dérivés de proline, comme le Sodium palmitoyl proline (Sepicalm VG de Seppic), ou le Mélange de Sodium palmitoyl sarcosinate, Magnesium palmitoyl glutamate, Palmitic acid et Palmitoyl proline (Sepifeel One de Seppic) ;
   - les lactylates, comme le Sodium stearoyl lactylate (Akoline SL de Karlshamns AB) ;
   - les sarcosinates, comme le sodium palmitoyl sarcosinate (Nikkol sarcosinate PN) ou le mélange de Stéaroyl sarcosine et Myristoyl sarcosine 75/25 (Crodasin SM de Croda) ;
   - les sulfonates, comme le Sodium C14-17 alkyl sec sulfonate (Hostapur SAS 60 de Clariant) ;
   - les glycinates, comme le sodium cocoyl glycinate (Amilite GCS-12 d'Ajinomoto) ;
b) les tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100^{®} par la société PHOENIX CHEMICAL.

### Tensioactifs additionnels

Le système émulsionnant peut en outre contenir au moins un agent tensioactif additionnel choisi de manière appropriée pour l'obtention d'une émulsion cire dans eau ou huile-dans-eau.
En particulier, on peut utiliser un émulsionnant possédant à 25 °C une balance HLB (hydrophile-lipophile balance) au sens de GRIFFIN, supérieure ou égale à 8.

Ces agents tensioactifs additionnels peuvent être choisis parmi les agents tensioactifs non ioniques, anioniques, cationiques, amphotères ou encore les émulsionnants tensioactifs. On peut se reporter au document "Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.

Ces tensioactifs additionnels peuvent être préférentiellement choisis parmi :
a) les tensioactifs non ioniques de HLB supérieur ou égal à 8 à 25 °C, utilisés seuls ou en mélange; on peut citer notamment :
   - les esters et éthers d'oses tels que le mélange de cétylstéaryl glucoside et d'alcools cétylique et stéarylique comme le Montanov 68 de Seppic;
   - les éthers oxyéthylénés et/ou oxypropylénés (pouvant comporter de 1 à 150 groupes oxyéthylénés et/ou oxypropylénés) de glycérol ;
   - les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA "Ceteareth-30"), l'éther oxyéthyléné de l'alcool stéarylique à 20 groupes oxyéthylénés (nom CTFA "Steareth-20"), et l'éther oxyéthyléné du mélange d'alcools gras en C12-C15 comportant 7 groupes oxyéthylénés (nom CTFA "C12-15 Pareth-7") commercialisé sous la dénomination NEODOL 25-7^{®} par SHELL CHEMICALS,
   - les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P^{®} par la société ICI UNIQUEMA,
   - les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM^{®} par la société SEPPIC ; le stéarate de glycéryle polyéthoxylé à 30 groupes d'oxyde d'éthylène comme le produit TAGAT S^{®} vendu par la société GOLDSCHMIDT, l'oléate de glycéryle polyéthoxylé à 30 groupes d'oxyde d'éthylène comme le produit TAGAT O^{®} vendu par la société GOLDSCHMIDT, le cocoate de glycéryle polyéthoxylé à 30 groupes d'oxyde d'éthylène comme le produit VARIONIC LI 13^{®} vendu par la société SHEREX, l'isostéarate de glycéryle polyéthoxylé à 30 groupes d'oxyde d'éthylène comme le produit TAGAT L^{®} vendu par la société GOLDSCHMIDT et le laurate de glycéryle polyéthoxylé à 30 groupes d'oxyde d'éthylène comme le produit TAGAT I^{®} de la société GOLDSCHMIDT,
   - les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 20 vendu sous la dénomination Tween 20^{®} par la société CRODA, le polysorbate 60 vendu sous la dénomination Tween 60^{®} par la société CRODA,
   - la diméthicone copolyol, telle que celle vendue sous la dénomination Q2-5220^{®} par la société DOW CORNING,
   - la diméthicone copolyol benzoate (FINSOLV SLB 101^{®} et 201^{®} de la société FINTEX),
   - les copolymères d'oxyde propylène et d'oxyde d'éthylène, également appelés polycondensats OE/OP,
   - et leurs mélanges.
   Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

   H-(O-CH₂-CH₂)ₐ-(O-CH(CH₃)-CH₂)_{b}-(O-CH₂-CH₂)ₐ-OH,

   formule dans laquelle a va de 2 à 120, et b va de 1 à 100.
   Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC^{®} comme les SYNPERONIC PE/ L44^{®} et SYNPERONIC PE/F127^{®} par la société ICI.
b) les tensioactifs non ioniques de HLB inférieur à 8 à 25 °C, éventuellement associés à un ou plusieurs agents tensioactif non ioniques de HLB supérieur à 8 à 25 °C, tels que cités ci-dessus tels que :
   - les esters et éthers d'oses tels que les stéarate de sucrose, cocoate de sucrose, stéarate de sorbitan et leurs mélanges comme l'Arlatone 2121^{®} commercialisé par la société ICI ;
   - les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool stéarylique à 2 groupes oxyéthylénés (nom CTFA "Steareth-2") ;
   - les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M^{®} par la société GOLDSCHMIDT, laurate de glycéryle tel que le produit vendu sous la dénomination IMWITOR 312^{®} par la société HULS, stéarate de polyglycéryl-2, tristéarate de sorbitan, ricinoléate de glycéryle ;
   - les lécithines, telles que les lécithines de soja (comme Emulmetik 100 J de Cargill, ou Biophilic H de Lucas Meyer) ;
   - le mélange de cyclométhicone/diméthicone copolyol vendu sous la dénomination Q2-3225C^{®} par la société DOW CORNING.
c) les tensioactifs anioniques tels que :
   - les sels d'acides gras en C16-C30 notamment ceux dérivant des amines, comme le stéarate de triéthanolamine et/ou le stéarate d'amino-2-méthyl-2-propane di-ol-1,3 ; mais de préférence la composition selon l'invention comprend moins 1% de stéarate de triéthanolamine et moins de 1% de stéarate d'amino-2-méthyl-2-propane di-ol-1,3.

Le tensioactif utilisable peut également être un tensioactif polymérique, notamment un polymère thermogélifiant.

Selon un mode de réalisation, la composition cosmétique selon la présente demande comprend moins de 1%, de préférence moins de 0,5% en poids de triéthanolamine, et mieux, est exempte de triéthanolamine. Selon un autre mode de réalisation, la composition cosmétique selon la présente demande comprend moins de 1%, de préférence moins de 0,5% en poids d'amino-2-méthyl-2-propane di-ol-1,3, et mieux, est exempte d'amino-2-méthyl-2-propane di-ol-1,3.

Selon une variante, la composition cosmétique selon la présente demande comprend moins de 1%, de préférence moins de 0,5% en poids de stéarate de triéthanolamine, et mieux, est exempte de stéarate de triéthanolamine. Selon une autre variante, la composition cosmétique selon la présente demande comprend moins de 1%, de préférence moins de 0,5% en poids de stéarate d'amino-2-méthyl-2-propane di-ol-1,3, et mieux, est exempte de stéarate d'amino-2-méthyl-2-propane di-ol-1,3.

Selon un mode de réalisation, la composition comprend en outre un co-tensioactif choisi parmi les alcools gras, comprenant de préférence de 10 à 30 atomes de carbone. Par alcool gras comprenant de 10 à 30 atomes de carbone, on entend tout alcool gras pur, saturé ou non, ramifié ou non, comportant de 10 à 30 atomes de carbone.
On utilise de préférence un alcool gras comprenant de 10 à 26 atomes de carbone, mieux de 10 à 24 atomes de carbone et encore mieux de 14 à 22 atomes de carbone.
On peut citer notamment comme alcools gras utilisables dans la composition les alcools laurique, myristique, cétylique, stéarylique, oléique, cétéarylique (mélange d'alcool cétylique et stéarylique), béhénique, érucique et leurs mélanges. On utilise de préférence l'alcool cétylique.
De tels alcools gras sont notamment commercialisés sous la dénomination NAFOL par la société SASOL.
L'alcool gras peut être présent en une teneur allant de 0,2 à 20% en poids, de préférence de 0,3 à 10% en poids par rapport au poids total de la composition.

De préférence la composition comprend le système tensioactif suivant :
- au moins un sel d'ester phosphorique comme tensioactif ionique présent dans la phase aqueuse sous forme de sel ;
- au moins un éther oxyéthyléné et/ou oxypropyléné, pouvant comporter de 1 à 150 groupes oxyéthylénés et/ou oxypropylénés, d'alcools gras en C12-C18, de HLB supérieur à 8 à 25 °C ;
- au moins un éther oxyéthyléné et/ou oxypropyléné, pouvant comporter de 1 à 150 groupes oxyéthylénés et/ou oxypropylénés, d'alcools gras C12-C18, de HLB inférieur à 8 à 25 °C,
- et éventuellement au moins un alcool gras ayant de 14 à 22 atomes de carbone,
à titre de système tensioactif principal de la composition.

Par « système tensioactif principal », on entend un système qui, en son absence, ne conduit pas à la formation d'une composition stable.

Par « stable », on entend une composition qui, après avoir été placée dans une étuve à 45°C pendant deux mois, ne présente pas, après retour à température ambiante, de grains perceptibles au toucher lorsqu'une couche fine de la composition est cisaillée entre les doigts.

Avantageusement, le système tensioactif décrit ci-dessus comme système tensioactif principal constitue l'unique système tensioactif de la composition.

Par « unique » on entend que tout éventuel système tensioactif additionnel est présent en une teneur n'excédant pas 1%, et de préférence n'excédant pas 0,5%. De préférence encore, par « unique » on désigne une absence totale de tout autre système tensioactif.

### Gélifiants hydrosolubles

La composition selon l'invention peut comprendre un gélifiant hydrosoluble.
Les gélifiants hydrosolubles utilisables dans les compositions selon l'invention peuvent être choisi parmi :
les homo- ou copolymères d'acides acrylique ou méthacrylique ou leurs sels et leurs esters et en particulier les produits vendus sous les dénominations VERSICOL F^{®} ou VERSICOL K^{®} par la société ALLIED COLLOID, UTRAHOLD 8^{®} par la société CIBA-GEIGY, les acides polyacryliques de type SYNTHALEN K,
les copolymères d'acide acrylique et d'acrylamide vendus sous la forme de leur sel de sodium sous les dénominations RETEN^{®} par la société HERCULES, le polyméthacrylate de sodium vendu sous la dénomination DARVAN N°7^{®} par la société VANDERBILT, les sels de sodium d'acides polyhydroxycarboxyliques vendus sous la dénomination HYDAGEN F^{®} par la société HENKEL,
les copolymères acide polyacryliques/acrylates d'alkyle de type PEMULEN,
l'AMPS (Acide polyacrylamidométhyl propane sulfonique neutralisé partiellement à l'ammoniaque et hautement réticulé) commercialisé par la société CLARIANT,
les copolymères AMPS/acrylamide de type SEPIGEL^{®} ou SIMULGEL^{®} commercialisés par la société SEPPIC, et
les copolymères AMPS/méthacrylates d'alkyle polyoxyéthylénés (réticulés ou non),
les protéines comme les protéines d'origine végétale telles que les protéines de blé, de soja ; les protéines d'origine animale tels que les kératines, par exemples les hydrolysats de kératine et les kératines sulfoniques ;
les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
les polymères ou copolymères acryliques, tels que les polyacrylates ou les polyméthacrylates ;
les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
les polymères d'origine naturelle, éventuellement modifiés, tels que :
   les gommes arabiques, la gomme de guar, les dérivés du xanthane, la gomme de karaya ;
   les alginates et les carraghénanes ;
   les glycoaminoglycanes, l'acide hyaluronique et ses dérivés ;
   la résine shellac, la gomme de sandaraque, les dammars, les élémis, les copals ;
   l'acide désoxyribonucléïque ;
   les muccopolysaccharides tels les chondroïtines sulfate,
   et leurs mélanges.

Certains de ces gélifiants hydrosolubles peuvent également jouer le rôle de polymères filmogènes.

Le polymère gélifiant hydrosoluble peut être présent dans la composition selon l'invention en une teneur en matières sèches allant de 0,01 % à 50 % en poids, de préférence de 0,5 % à 30 % en poids, mieux de 1 % à 20 % en poids, voire de 2 à 10 % en poids par rapport au poids total de la composition.

### Cires polaires

La composition selon la présente demande comprend au moins une cire polaire. Cette cire polaire est indispensable pour obtenir des textures épaisses et chargeantes.
Par cire, on entend un composé lipophile, solide à température ambiante (25 °C), déformable ou non, à changement d'état solide/liquide réversible, ayant un point de fusion supérieur ou égal à 40 °C pouvant aller jusqu'à 120 °C. En particulier, les cires convenant à l'invention peuvent présenter un point de fusion supérieur ou égal à 45 °C, et en particulier supérieur ou égal à 55°C.
Par composé lipophile, on entend un composé ayant un indice d'acide et un indice d'hydroxyle inférieur à 150 mg KOH/g.
Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.
Le protocole de mesure est le suivant :
Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires polaires peuvent être hydrocarbonées, et/ou fluorées et être d'origine végétale, minérale, animale et/ou synthétique.
Par « cire polaire », on entend des cires comprenant dans leur structure chimique, en plus des atomes de carbone et d'hydrogène, au moins un hétéroatome fortement électronégatif, tels que O, N ou P.

La cire polaire peut être présente en une teneur allant de 1 à 50 % en poids par rapport au poids total de la composition, mieux de 2 à 40 % et encore mieux de 5 à 30% en poids.

On peut notamment utiliser comme cire polaire les cires hydrocarbonées comme la cire d'abeille, la cire de lanoline; la cire de riz, la cire de Carnauba, la cire de Candellila, la cire d'Ouricurry, la cire du Japon, la cire de Berry, la cire de shellac et la cire de sumac; la cire de montan.
Selon un mode préféré, on utilisera une cire hydrocarbonée choisie parmi la cire d'abeille, la cire de son de riz, la cire de Carnauba, et leurs mélanges.
On peut aussi citer les cires obtenues par hydrogénation catalytique d'huiles animales ou végétales ayant des chaînes grasses, linéaires ou ramifiées, en C8-C32.
Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination "HEST 2T-4S" par la société HETERENE, le tétrabéhénate de di-(triméthylol-1,1,1 propane) vendue sous la dénomination HEST 2T-4B par la société HETERENE.
On peut également utiliser la cire obtenue par hydrogénation d'huile d'olive estérifiée avec l'alcool stéarylique vendue sous la dénomination "PHYTOWAX Olive 18 L 57" ou bien encore les cires obtenues par hydrogénation d'huile de ricin estérifiée avec l'alcool cétylique vendus sous la dénomination "PHYTOWAX ricin 16L64 et 22L73", par la société SOPHIM. De telles cires sont décrites dans la demande FR-A-2792190.
On peut également citer les cires issues de la réaction d'acides gras sur des carbohydrates, comme les disaccharides de type sucrose, telles que le polybéhénate de sucrose, commercialisé par Croda sous la référence Cromaderm B.

Selon un mode de réalisation particulier, les compositions conformes à l'invention peuvent comprendre au moins une cire dite cire collante c'est-à-dire possédant un collant supérieur ou égal à 0,7 N.s et une dureté inférieure ou égale à 3,5 MPa.
L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.
La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.
Le collant de la cire est déterminé par la mesure de l'évolution de la force (force de compression ou force d'étirement) en fonction du temps, à 20 °C à l'aide du texturomètre vendu sous la dénomination "TA-TX2i®" par la société RHEO, équipé d'un mobile en polymère acrylique en forme de cône formant un angle de 45°.
Le protocole de mesure est le suivant :
La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.
Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.
La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.
La dureté est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.
Le protocole de mesure est le suivant :
La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.
Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.
La valeur de la dureté est la force de compression maximale mesurée divisée par la surface du cylindre du texturomètre en contact avec la cire.

Comme cire collante, on peut utiliser un (hydroxystéaryloxy)stéarate d'alkyle en C20-C40 (le groupe alkyle comprenant de 20 à 40 atomes de carbone), seul ou en mélange, en particulier un 12-(12'-hydroxystéaryloxy)stéarate d'alkyle en C20-C40, de formule (II) : 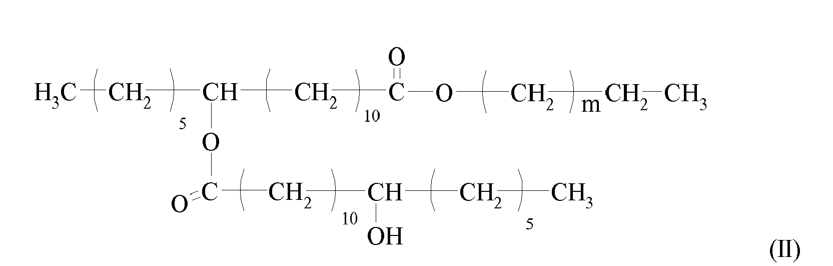 dans laquelle m est un entier allant de 18 à 38, ou un mélange de composés de formule (II).
Une telle cire est notamment vendue sous les dénominations "Kester Wax K 82 P®" et "Kester Wax K 80 P®" par la société KOSTER KEUNEN.
Les cires citées ci-dessus présentent généralement un point de fusion commençante inférieur à 45 °C.

La ou les cires peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 µm.
Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.
Les microdispersions de cire peuvent également être obtenues par agitation du mélange de cire, de tensioactif et d'eau à l'aide de moyen d'agitation tels que les ultrasons, l'homogénéisateur haute pression, les turbines.
Les particules de la microdispersion de cire ont de préférence des dimensions moyennes inférieures à 1 µm (notamment allant de 0,02 µm à 0,99 µm), de préférence inférieures à 0,5 µm (notamment allant de 0,06 µm à 0,5 µm).
Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

On peut citer notamment les micro cires de cire synthétique telles que celle commercialisée sous la dénomination de « MicroEase 114S^{®} » par la société MICRO POWDERS.

De préférence, les cires polaires sont choisies parmi les cires hydrocarbonées comme la cire d'abeille, la cire de lanoline ; la cire de son de riz, la cire de Carnauba, la cire de Candellila, la cire d'Ouricurry, la cire du Japon, la cire de Berry, la cire de shellac et la cire de sumac; la cire de Montan, l'huile de ricin hydrogénée, l'huile de lanoline hydrogénée, les cires issues de la réaction d'acides gras sur des carbohydrates, comme les disaccharides de type sucrose, telles que le polybéhénate de sucrose, commercialisé par Croda sous la référence Cromaderm B, les cires hydroxyesters comme par exemple les cires (hydroxystéaryloxy)stéarate d'alkyle en C20-C40 telles que celles vendues sous les dénominations "Kester Wax K 82 P®" et "Kester Wax K 80 P®" par la société KOSTER KEUNEN.
De préférence encore, les cires polaires sont choisies parmi les cires hydrocarbonées comme la cire d'abeille, la cire de son de riz, la cire de Carnauba, les cires collantes, c'est-à-dire possédant un collant supérieur ou égal à 0,7 N.s et une dureté inférieure ou égale à 3,5 MPa, les cires collantes étant de préférence les cires hydroxyesters, de préférence les cires (hydroxystéaryloxy)stéarate d'alkyle en C20-C40 telles que celles vendues sous les dénominations "Kester Wax K 82 P®" et "Kester Wax K 80 P®" par la société KOSTER KEUNEN, et leurs mélanges.

### Cires additionnelles

La composition selon l'invention peut également comprendre une ou plusieurs cires additionnelles distinctes de la cire polaire listée ci-dessus. De telles cires additionnelles sont des cires apolaires.
La cire apolaire considérée dans le cadre de la présente invention répond à la définition de la cire donnée ci-dessus.
Par « apolaire », on entend des cires ne comprenant pas, dans leur structure chimique, d'hétéroatomes fortement électronégatifs, tels que O, N ou P.

Les cires apolaires peuvent être hydrocarbonées, fluorées et être d'origine minérale et/ou synthétique.
La cire apolaire peut être présente en une teneur allant de 1 à 50 % en poids par rapport au poids total de la composition, mieux de 1 à 40 % et encore mieux de 1 à 10% en poids.

A titre illustratif des cires apolaires convenant à l'invention, on peut notamment citer les cires microcristallines, les paraffines et l'ozokérite; les cires de polyéthylène, les cires obtenues par la synthèse de Fisher-Tropsch et les copolymères cireux ainsi que leurs esters ; les cires de silicone et les cires fluorées.
Selon un mode particulier, on utilise comme cire additionnelle, les paraffines.

### Huiles

La composition selon l'invention peut en outre comprendre une ou plusieurs huiles ou corps gras non aqueux liquides à température ambiante (25°C) et pression atmosphérique (760 mm de Hg).
L'huile peut être choisie parmi les huiles volatiles et/ou les huile non volatiles, et leurs mélanges.

La ou les huiles peuvent être présentes dans la composition selon l'invention en une teneur allant de 0,1 % à 30 % en poids, de préférence de 0,5 % à 20 % en poids par rapport au poids total de la composition.

Par " huile volatile", on entend au sens de l'invention une huile susceptible de s'évaporer au contact des fibres kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa (10⁻³ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg). Par "huile non volatile", on entend une huile restant sur la fibre kératinique à température ambiante et pression atmosphérique au moins plusieurs heures et ayant notamment une pression de vapeur inférieure à 10⁻³ mm de Hg (0,13 Pa).
Ces huiles peuvent être des huiles hydrocarbonées, des huiles siliconées, des huiles fluorées, ou leurs mélanges.
On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.
Comme huiles volatiles, on peut aussi utiliser les silicones volatiles, comme par exemple les huiles de silicones linéaires ou cycliques volatiles, notamment celles ayant une viscosité ≤ 8 centistokes (8 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.
On peut également citer les huiles linéaires alkyltrisiloxanes volatiles de formule générale (I) : 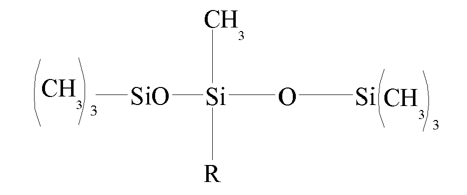 où R représente un groupe alkyle comprenant de 2 à 4 atomes de carbone et dont un ou plusieurs atomes d'hydrogène peuvent être substitués par un atome de fluor ou de chlore. Parmi les huiles de formule générale (I), on peut citer :
le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.
On peut également utiliser des solvants volatils fluorés tels que le nonafluorométhoxybutane ou le perfluorométhylcyclopentane.

La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :
- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- les carbonates,
- les acétales,
- les citrates,
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

### Polymères filmogènes

La composition selon l'invention peut comprendre, outre le copolymère comprenant au moins un monomère alcène, au moins un polymère filmogène.

Le polymère filmogène peut être présent dans la composition selon l'invention en une teneur en matières sèches (ou matières actives) allant de 0,1 % à 30 % en poids par rapport au poids total de la composition, de préférence de 0,5 % à 20 % en poids, et mieux de 1 % à 15 % en poids.

Dans la présente invention, on entend par « polymère filmogène », un polymère apte à former à lui seul ou en présence d'un agent auxiliaire de filmification, un film macroscopiquement continu et adhérent sur les fibres kératiniques.

Le polymère filmogène hydrophile peut être un polymère hydrosoluble ou se présenter en dispersion dans un milieu aqueux.
Parmi les polymères filmogènes utilisables dans la composition de la présente invention, on peut citer les polymères synthétiques, de type radicalaire ou de type polycondensat, les polymères d'origine naturelle, et leurs mélanges.
Par polymère filmogène radicalaire, on entend un polymère obtenu par polymérisation de monomères à insaturation notamment éthylénique, chaque monomère étant susceptible de s'homopolymériser (à l'inverse des polycondensats).

Comme exemples de polymères filmogènes hydrosolubles, on peut citer :
- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères de chitine ou de chitosane anioniques, cationiques, amphotères ou non-ioniques,
- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels les chondroïtines sulfate,
et leurs mélanges.

Le polymère filmogène peut être également présent dans la composition sous la forme de particules en dispersion dans une phase aqueuse, connue généralement sous le nom de latex ou pseudolatex. Les techniques de préparation de ces dispersions sont bien connues de l'homme du métier.
Comme dispersion aqueuse de polymère filmogène, on peut utiliser les dispersions acryliques vendues sous les dénominations Neocryl XK-90^{®}, Neocryl A-1070^{®}, Neocryl A-1090^{®}, Neocryl BT-62^{®}, Neocryl A-1079^{®} et Neocryl A-523^{®} par la société AVECIA-NEORESINS, Dow Latex 432^{®} par la société DOW CHEMICAL, Daitosol 5000 AD^{®} ou Daitosol 5000 SJ^{®} par la société DAITO KASEY KOGYO; Syntran 5760^{®} par la société Interpolymer Allianz Opt^{®} par la société Rohm and Haas ou encore les dispersions aqueuses de polyuréthane vendues sous les dénominations Neorez R-981^{®} et Neorez R-974^{®} par la société AVECIA-NEORESINS, les Avalure UR-405^{®}, Avalure UR-410^{®}, Avalure UR-425^{®}, Avalure UR-450^{®}, Sancure 875^{®}, Avalure UR-445^{®} et Sancure 2060^{®} par la société NOVEON, Impranil 85^{®} par la société BAYER, Aquamere H-1511^{®} par la société HYDROMER; les sulfopolyesters vendus sous le nom de marque Eastman AQ^{®} par la société Eastman Chemical Products, les dispersions vinyliques comme le Mexomère PAM^{®}, les dispersions aqueuses de polyvinyl acétate comme le "Vinybran^{®}" de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références "Hybridur^{®}" par la société AIR PRODUCTS ou "Duromer^{®}" de NATIONAL STARCH, les dispersions type core/shell : par exemple celles commercialisées par la société ATOFINA sous la référence Kynar (core : fluoré-shell : acrylique) ou encore ceux décrits dans le document US 5 188 899 (core ; silice - shell : silicone) et leurs mélanges.

Selon une autre variante de réalisation de la composition selon l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). De préférence, la phase grasse liquide comprend une huile volatile, éventuellement en mélange avec une huile non volatile, les huiles pouvant être choisies parmi les huiles citées précédemment.
A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).
Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.
Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécylvinyléther, propionate de vinyle/laurate d'allyle, propionate de vinyle/laurate de vinyle, stéarate de vinyle/octadécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.
Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.
De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.
Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.
Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.
A titre d'exemples de résines polymethylsilsesquioxanes commercialement disponibles, on peut citer celles qui sont commercialisés :
par la société Wacker sous la référence Resin MK tels que la Belsil PMS MK :
   par la société SHIN-ETSU sous les références KR-220L.
Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.
On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction d'un résine de silicone, telle que celles décrite plus haut, et d'un diorganosiloxane tels que décrits dans le document WO 2004/073626.

Le polymère filmogène peut être également présent dans la composition sous la forme de particules en dispersion dans une phase non aqueuse. Comme exemples de dispersions non aqueuses de polymère filmogène, on peut citer les dispersions acryliques dans l'isododécane comme le Mexomère PAP^{®} de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

La composition peut également comprendre des ingrédients couramment utilisés en cosmétique tels que les matières colorantes, les charges, les fibres et leurs mélanges.

### Matière colorante

La composition selon l'invention peut également comprendre au moins une matière colorante comme les matières pulvérulentes, les colorants liposolubles, les colorants hydrosolubles.
Les matières colorantes pulvérulentes peuvent être choisies parmi les pigments et les nacres.
Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.
Ces matières colorantes peuvent être présentes en une teneur allant de 0,01 à 30 % en poids par rapport au poids total de la composition.

### Charges

La composition selon l'invention peut en outre comprendre au moins une charge.
Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon^{®} commercialisé sous la dénomination Orgasol^{®} par la société Atochem, de poly-β-alanine et de polyéthylène, les poudres de polymères de tétrafluoroéthylène comme le Téflon^{®}, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel^{®} par la société Nobel Industrie, les poudres acryliques telles que celles commercialisées sous la dénomination Polytrap^{®} par la société Dow Corning, les particules de polyméthacrylate de méthyle et les microbilles de résine de silicone (Tospearls^{®} de Toshiba, par exemple), le carbonate de calcium précipité, le carbonate et l'hydro-carbonate de magnésium, l'hydroxyapatite, les microsphères de silice creuses (Silica Beads^{®} de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.
On peut également utiliser un composé susceptible de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel^{®} 820 DU 40 et Expancel^{®}007WU par la Société AKZO NOBEL.
Les charges peuvent représenter de 0,1 à 25 %, en particulier de 1 à 20 % en poids par rapport au poids total de la composition.

### Fibres

Les compositions conformes à l'invention peuvent également comprendre au moins une fibre qui permet une amélioration de l'effet allongeant.
Par "fibre", il faut comprendre un objet de longueur L et de diamètre D tel que L soit très supérieur à D, D étant le diamètre du cercle dans lequel s'inscrit la section de la fibre.
En particulier, le rapport L/D (ou facteur de forme) est choisi dans la gamme allant de 3,5 à 2500, en particulier de 5 à 500, et plus particulièrement de 5 à 150.
Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.
En particulier, les fibres ont une longueur allant de 1 µm à 10 mm, en particulier de 0,1 mm à 5 mm et plus particulièrement de 0,3 mm à 3,5 mm. Leur section peut être comprise dans un cercle de diamètre allant de 2 nm à 500 µm, en particulier allant de 100 nm à 100 µm et plus particulièrement de 1 µm à 50 µm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.
Les fibres utilisables dans la composition de l'invention peuvent être choisies parmi les fibres rigides ou non rigides, elles peuvent être d'origine synthétique ou naturelle, minérales ou organiques.
Par ailleurs, les fibres peuvent être traitées ou non en surface, enrobées ou non, colorées ou non colorées.
A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénominations KERMEL^{®}, KERMEL TECH^{®} par la société RHODIA ou de poly-(p-phénylène-téréphtalamide) (ou d'aramide) notamment vendues sous la dénomination Kevlar ® par la société DUPONT DE NEMOURS.
Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

### Actifs cosmétiques

Comme actifs cosmétiques pouvant être utilisés dans les compositions selon l'invention, on peut citer notamment des antioxydants, des conservateurs, des parfums, des neutralisants, des émollients, des hydratants, des vitamines et des filtres en particulier solaires.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

La composition peut se présenter sous forme solide, semi-solide ou liquide.
La composition peut se présenter notamment sous forme de suspension, de dispersion, de solution, de gel, d'émulsion, notamment émulsion huile-dans-eau (H/E), cire-dans-eau, ou multiple (E/H/E ou polyol/H/E) sous forme de crème, de pâte, de mousse, de dispersion de vésicules notamment de lipides ioniques ou non, de lotion biphase ou multiphase, de spray, de poudre, de pâte, notamment de pâte souple. Chaque composition est de préférence une composition non rinçée.

La composition selon l'invention peut être fabriquée par les procédés connus, généralement utilisés dans le domaine cosmétique.

La composition selon l'invention peut être conditionnée dans un récipient délimitant au moins un compartiment qui comprend ladite composition, ledit récipient étant fermé par un élément de fermeture.

Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).

Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

Les exemples figurant ci-après sont présentés à titre illustratif et non limitatif de l'invention. Sauf indication contraire, les quantités indiquées sont exprimées en pourcentage massique.

### Exemple 1 : Mascara

| | **Composition 1** | **Exemple Comparatif** |
|---|---|---|
| Copolymère Ethylène/Acétate de Vinyl (28% Vinyl Acetate, Mw=70000) | 7,2 | 0,0 |
| Cire de Paraffine | 5,6 | 12,8 |
| Cire de carnauba | 5,0 | 5,0 |
| Cire abeille | 4,1 | 4,1 |
| Alcool cétylique | 2,0 | 2,0 |
| Stéareth 20 | 4,4 | 4,4 |
| Stéareth 2 | 2,1 | 2,1 |
| Oxyde de fer noir | 7,1 | 7,1 |
| Glycérine | 2,0 | 2,0 |
| Butylène glycol | 3,0 | 3,0 |
| Potassium cetyl Phosphate | 1,7 | 1,7 |
| Hydroxyethylcellulose | 0,9 | 0,9 |
| Gomme arabique | 3,4 | 3,4 |
| eau | Qs 100 | Qs 100 |

Les cires, le copolymère, l'alcool cétylique, le steareth 2 et le stéareth 20 sont fondus à 85°C. Le pigment oxyde de fer noir est ensuite dispersé dans cette phase grasse sous forte agitation. Dans un bécher secondaire, la phase aqueuse contenant le potassium cetylphosphate est mélangée et chauffée à 85 °C.
L'émulsion est alors réalisée sous forte agitation en ajoutant la phase aqueuse, également chauffée à 85 °C sur la phase grasse. L'ensemble est ensuite refroidi sous pales jusqu'à température ambiante.

La composition 1 et l'exemple comparatif sont évalués du point de vue application, adhérence et charge par un panel de 10 experts. Des scores allant de 0 à 10 sont attribués pour chaque paramètre, le score 0 étant attribué pour une performance nulle, et le score 10 pour une performance maximale.
La composition 1 est jugée significativement moins sèche à l'application, plus adhérente instantanément, plus chargeante que l'exemple comparatif.

Par ailleurs, la composition 1 selon l'invention conduit à un produit plus intense en noir que l'exemple comparatif.

### Exemple 2 : Mascara

| | **Composition 2** | **Exemple Comparatif** |
|---|---|---|
| Copolymère Ethylène/Acétate de Vinyl (28% Vinyl Acetate, Mw=70000) | 2,4 | 2,4 |
| Cire de Paraffine | 10,5 | 10,5 |
| Cire de carnauba | 5,0 | 5,0 |
| Cire abeille | 4,1 | 4,1 |
| Alcool cétylique | 2,0 | 2,0 |
| Acide Stéarique | 0 | 6.6 |
| Stéareth 20 | 4,4 | 0 |
| Stéareth 2 | 2,1 | 0 |
| Oxyde de fer noir | 7,1 | 7,1 |
| Glycérine | 2,0 | 2,0 |
| Butylène glycol | 3,0 | 3,0 |
| Potassium cetyl Phosphate | 1,7 | 0 |
| Triéthanol Amine | 0 | 2.4 |
| AMPD | 0 | 0.5 |
| Hydroxyethylcellulose | 0,9 | 0,9 |
| Gomme arabique | 3,4 | 3,4 |
| Ethanol | 3 | 3 |
| eau | Qs 100 | Qs 100 |

La composition 2 comprend un tensioactif ionique selon l'invention, présent sous forme de sel dans la phase aqueuse, ie le potassium cétyl phosphate, tandis que l'exemple comparatif comprend un système tensioactif classique, ie la combinaison acide stéarique/triéthanolamine.
Il ressort que la composition 2 obtenue est souple et lisse, et se maquille très bien à l'aide d'une brosse de mascara, alors que l'exemple comparatif donne une pâte granuleuse, très épaisse et difficile à maquiller.

### Exemple 3 : Mascara contenant des fibres

| | **Composition 3** |
|---|---|
| Copolymère Ethylène/Acétate de Vinyl (28% Vinyl Acetate, Mw=70000) | 3,3 |
| Cire de Paraffine | 12,5 |
| Cire de carnauba | 5,6 |
| Cire abeille | 4,5 |
| Alcool cétylique | 2,0 |
| Stéareth 20 | 4,4 |
| Stéareth 2 | 2,1 |
| Oxyde de fer noir | 7,1 |
| Glycérine | 2,0 |
| Butylène glycol | 3,0 |
| Potassium cetyl Phosphate | 1,7 |
| Hydroxyethylcellulose | 0,9 |
| Gomme arabique | 3,4 |
| Fibre de cellulose (Rayonne) | 0.5 |
| eau | Qs 100 |

### Exemple 4 : Mascara

| | **Composition 4** | **Exemple Comparatif** |
|---|---|---|
| Copolymère Styrène-Ethylène/Butylène-Styrène1 (Kraton G1657M de Shell, Mw=130000) | 2,75 | 0 |
| Cire de Paraffine | 11,25 | 14,0 |
| Cire de carnauba | 3,5 | 3,5 |
| Cire abeille | 4,4 | 4,4 |
| Alcool cétylique | 2,0 | 2,0 |
| Stéareth 2 | 2,1 | 2,1 |
| Oxyde de fer noir | 7,1 | 7,1 |
| Potassium cetyl Phosphate | 7,0 | 7,0 |
| Hydroxyethylcellulose | 0,75 | 0,75 |
| Gomme arabique | 0,65 | 0,65 |
| Copolymère acrylate en dispersion aqueuse (Daitosol 5000 AD de Daito) | 10,0 | 10,0 |
| eau | Qs 100 | Qs 100 |

Les cires, le copolymère, l'alcool cétylique, le steareth 2 sont fondus à 85°C. Le pigment oxyde de fer noir est ensuite dispersé dans cette phase grasse sous forte agitation. Dans un bécher secondaire, la phase aqueuse contenant le potassium cetylphosphate, la gomme arabique et l'hydroxycellulose est mélangée et chauffée à 85 °C.
L'émulsion est alors réalisée sous forte agitation en ajoutant la phase aqueuse, également chauffée à 85 °C sur la phase grasse. L'ensemble est ensuite refroidi sous pales jusqu'à température ambiante pour ajouter le copolymère acrylate en solution aqueuse..

La composition 4 conduit à un produit plus intense en noir que l'exemple comparatif.

## Revendications

1. Composition cosmétique de maquillage ou de soin non thérapeutique des fibres kératiniques comprenant une phase aqueuse continue, au moins un composé choisi parmi les copolymères comprenant au moins un monomère alcène, au moins une cire polaire et au moins un tensioactif ionique présent dans la phase aqueuse sous forme de sel.

2. Composition selon la revendication 1, **caractérisée en ce que** le composé choisi parmi les copolymères comprenant au moins un monomère alcène est choisi parmi :
- les copolymères d'alcène et d'acétate de vinyle, de préférence les copolymères d'éthylène et d'acétate de vinyle ;
- les copolymères d'α-oléfine linéaire ou ramifiée, en particulier d'α-oléfine en C₂-C₁₆ et mieux en C₂-C₁₂
- les copolymères d'éthylène et d'octène ;
- les copolymères d'éthylène ou de propylène et d'une cyclooléfine ;
- les copolymères à blocs (dibloc, tribloc, multibloc, radial, étoile) styrène et à blocs éthylène/alkylène en C₃-C₄ ;
- et leurs mélanges.

3. Composition selon l'une des revendications précédentes, **caractérisée en ce que** le composé choisi parmi les copolymères comprenant au moins un monomère alcène est choisi parmi les copolymère d'éthylène et d'acétate de vinyle comprenant entre 20 et 40% en poids d'acétate de vinyle par rapport au poids total du polymère.

4. Composition selon l'une des revendications précédentes, **caractérisée en ce que** le composé choisi parmi les copolymères comprenant au moins un monomère alcène est présent en une teneur en matières sèches supérieure ou égale à 1% en poids par rapport au poids total de la composition.

5. Composition selon l'une des revendications 1 à 3, **caractérisée en ce que** le composé choisi parmi les copolymères comprenant au moins un monomère alcène est présent en une teneur en matières sèches allant de 0,5 à 20% en poids par rapport au poids total de la composition, de préférence allant de 1 à 10% en poids.

6. Composition selon l'une des revendications précédentes, **caractérisée en ce que** la cire polaire est choisie parmi la cire d'abeille, la cire de lanoline, la cire de son de riz, la cire de Carnauba, la cire de Candellila, la cire d'Ouricurry, la cire du Japon, la cire de Berry, la cire de shellac, la cire de sumac, la cire de Montan, l'huile de ricin hydrogénée, l'huile de lanoline hydrogénée, les cires issues de la réaction d'acides gras sur des carbohydrates et les cires (hydroxystéaryloxy)stéarate d'alkyle en C20-C40.

7. Composition selon l'une des revendications précédentes, **caractérisée en ce que** la cire polaire est présente en quantité comprise entre 1 et 50% en poids par rapport au poids total de la composition, de préférence comprise entre 2 et 40% en poids.

8. Composition selon l'une des revendications précédentes, **caractérisée en ce que** la phase aqueuse est présente en quantité au moins égale à 30% en poids par rapport au poids total de la composition, de préférence au moins égale à 40% en poids.

9. Composition selon l'une des revendications précédentes, **caractérisée en ce que** le tensioactif ionique présent dans la phase aqueuse sous forme de sel est choisi parmi :
- les sels d'acides gras polyoxyéthylénés, et leurs mélanges ;
- les esters phosphoriques et leurs sels ;
- les sulfosuccinates ;
- les alkyléthersulfates ;
- les iséthionates ;
- les acylglutamates et leurs mélanges ;
- les dérivés de soja ;
- les citrates ;
- les dérivés de proline ;
- les lactylates ;
- les sarcosinates ;
- les sulfonates ;
- les glycinates ;
- les N-acyl-aminoacides ;
- les oxydes d'amines ;
- les diméthicone copolyols phosphates.

10. Composition selon l'une des revendications précédentes, **caractérisée en ce que** le tensioactif ionique présent dans la phase aqueuse sous forme de sel est présent en quantité comprise entre 0,1 et 20% en poids, de préférence entre 0,3 et 15% en poids par rapport au poids total de la composition.

11. Composition selon l'une des revendications précédentes, **caractérisée en ce qu'**elle comprend à titre de système tensioactif principal :
- au moins un sel d'ester phosphorique ;
- au moins un éther oxyéthyléné et/ou oxypropyléné, pouvant comporter de 1 à 150 groupes oxyéthylénés et/ou oxypropylénés, d'alcools gras en C12-C18, de HLB supérieur à 8 à 25°C ;
- au moins un éther oxyéthyléné et/ou oxypropyléné, pouvant comporter de 1 à 150 groupes oxyéthylénés et/ou oxypropylénés, d'alcools gras C12-C18, de HLB inférieur à 8 à 25°C,
- et éventuellement au moins un alcool gras ayant de 14 à 22 atomes de carbone.

12. Composition selon l'une des revendications précédentes, **caractérisée en ce qu'**elle comprend moins de 1%, de préférence moins de 0,5% en poids de triéthanolamine, et mieux, est exempte de triéthanolamine.

13. Composition selon l'une des revendications précédentes, **caractérisée en ce qu'**elle comprend moins de 1%, de préférence moins de 0,5% en poids d'amino-2-méthyl-2-propane di-ol-1,3, et mieux, est exempte d'amino-2-méthyl-2-propane di-ol-1,3

14. Procédé cosmétique de maquillage ou de soin non thérapeutique des fibres kératiniques comprenant l'application sur les fibres kératiniques d'une composition cosmétique selon l'une quelconque des revendications précédentes.

15. Ensemble de conditionnement d'une composition de maquillage et/ou de soin des fibres kératiniques comprenant :
i) un récipient délimitant au moins un compartiment ;
ii) une composition de maquillage et/ou de soin des fibres kératiniques disposée à l'intérieur dudit compartiment, ladite composition étant conforme à l'une des revendications 1 à 13.
